# FASCICULE DE BREVET EUROPEEN

(11) **EP 1 102 543 B1**
(45) Date de publication et mention de la délivrance du brevet: **30.08.2006**
(21) Numéro de dépôt: 99938342.5
(22) Date de dépôt: 20.07.1999
(51) Int. Cl.: A23C 9/12

(54) **PRODUIT LAITIER ANTI-CARIOGENE ET SON UTILISATION**
ANTIKARIOGENISCHES MILCHPRODUKT UND DESSEN VERWENDUNG
ANTI-CARIOGENIC MILK PRODUCT AND USE

(30) Priorité: 07.08.1998 EP 98202658
(43) Date de publication de la demande: 30.05.2001
(73) Titulaire: Société des Produits Nestlé S.A., 1800 Vevey (CH)
(72) Inventeur: NEESER, Jean-Richard, CH-1073 Savigny (CH); GUGGENHEIM, Bernhard, CH-8703 Erlenbach ZH (CH); PARMANTIER, Claude, F-14100 Glos (FR)
(86) Numéro de dépôt international: PCT/EP1999/005335
(87) Numéro de publication internationale: WO 2000/007454

(56) Documents cités:
- EP-A- 0 117 321
- EP-A- 0 283 675
- EP-A- 0 604 802
- DE-A- 2 232 641
- FR-A- 1 241 283
- FR-A- 1 545 635
- US-A- 2 376 693
- US-A- 2 928 742
- US-A- 5 368 869
- DATABASE WPI Section Ch, Week 7635 Derwent Publications Ltd., London, GB; Class D13, AN 76-65572X XP002090247 & JP 51 079762 A (LOTTE CO LTD), 12 juillet 1976 (1976-07-12)
- HARPER D S ET AL: "Cariostatic evaluation of cheeses with diverse physical and compositional characteristics." CARIES RESEARCH, vol. 20, no. 2, 1986, pages 123-130, XP002090244 American Dental Ass., Health Foundation Res. Inst., Chicago, Illinois, USA
- DATABASE FSTA [Online] INTERNATIONAL FOOD INFORMATION SERVICE (IFIS), FRANFURT/MAIN, DE AN 85-1-11-p0042, OMAR M M: "Size distribution of casein micelles during milk coagulation." XP002090246 & NAHRUNG, vol. 29, no. 2, 1985, pages 119-124, Food Sci. Dep., Univ. of Zagazig, Zagazig, Egypt

## Description

La présente invention concerne un produit laitier qui possède des propriétés anti-cariogènes. La présente invention concerne également les compositions alimentaires préparées à partir dudit produit.

### Etat de la technique

Certains produits dérivés du lait ont un grand intérêt pour la santé bucco-dentaire. Ainsi, on reconnaît à la caséine sous certaines formes et à certains de ses dérivés des propriétés anti-cariogènes. Quelques fromages, par exemple, sont connus pour leur activité anti-carie (Nutrition Reviews, 46, 215-217, 1988; Pause, B. and Lembke, J., Milchwissenschaft, 47, 697-700, 1992; ; Pause, B. and Lembke, J., Milchwissenschaft, 48, 137-141, 1993).

Dans le brevet US 5427769 (Nestec S.A.) on empêche l'apparition des caries dentaires en plaçant au contact des dents une composition alimentaire contenant de la caséine micellaire en quantité suffisante pour inhiber la colonisation par *Streptococcus sobrinus,* l'agent bactérien principalement impliqué dans la pathologie carieuse.

Le brevet EP 748591 (Société des Produits Nestlé S.A.) décrit également l'utilisation de caséines micellaires fluorées ou leurs sous unités micellaires pour traiter la plaque ou les caries dentaires.

Le brevet US 4992420 (Nestec S.A.) décrit le traitement de la cavité buccale avec des K-caséino-glycomacropeptides (CGMP) dérivés de petit-lait pour éradiquer la plaque dentaire et les caries. De même, le brevet US 4994441 (Nestec S.A.) décrit une composition anti-plaque et anti-carie dans laquelle l'agent actif est choisi parmi les κ-caséinoglycopeptides.

Aussi, le brevet US 5,015,628 (University of Melbourne) décrit une composition anti-cariogène contenant comme agent actif en particulier des phosphopeptides obtenus, par exemple, par hydrolyse trypsique des caséines α et β.

Tous ces documents décrivent des procédés qui n'appartiennent pas à la technologie des produits laitiers frais. Les procédés actuellement mis en oeuvre pour fabriquer de tels produits frais conduisent à des produits acides, tels que le yaourt, le fromage frais ou encore le "petit suisse". Dans de tels produits, la coagulation acide de la caséine conduit à sa décalcification, entraînant ainsi la perte d'une partie de ses propriétés anti-cariogènes. De même le CGMP contenu dans ces produits n'est pas hydrolysé, et de ce fait n'est pas sous une forme active.

La présente invention se propose de palier ces inconvénients, en proposant un produit laitier frais ayant des propriétés anti-cariogènes optimalisées.

### Résumé de l'invention

La présente invention a pour principal objet l'utilisation d'un lait "emprésuré" pour préparer des compositions alimentaires réfrigérées destinées à prévenir ou à traiter la carie dentaire ou la plaque dentaire. Ce lait emprésuré est préparé en coagulant du lait à pH neutre avec de la présure, puis en ajoutant un sel de calcium. Le lait emprésuré est stabilisé sous forme de gel. Il comprend du CGMP libre et de la caséine toujours complexée avec du calcium (caséine calcifiée), bien qu'ayant perdu sa forme micellaire.

On a pu en effet constater que ce lait coagulé ne possède plus une caséine micellaire, mais que celle-ci garde néanmoins une partie de ses propriétés, notamment quant à sa calcification. De plus, le sérum n'étant pas séparé du coagulum, le CGMP est conservé avec le produit.

Des résultats in-vivo montrent que ce lait a des propriétés anti-cariogènes supérieures à celles d'autres produits laitiers réfrigérés tel que le yaourt, par exemple. L'avantage de l'invention par rapport aux produits déjà connus, est de fournir un produit laitier frais dont l'activité est optimalisée et qui rassemble sous leur forme active, deux constituants anti-caries du lait, à savoir la caséine calcifiée et le CGMP.

Enfin, la présente invention a pour objet les compositions alimentaires préparées à partir de ce lait emprésuré, lequel est ajouté en quantité efficace de manière à obtenir un produit laitier frais ayant une activité anti-carie et anti-plaque dentaire optimalisée. Ces compositions peuvent également se présenter sous la forme d'une poudre.

### Description détaillée

Dans la description, les pourcentages sont indiqués en poids par rapport au poids total, sauf indication contraire.

Le lait emprésuré selon la présente invention est utilisé pour préparer des compositions alimentaires destinées à prévenir ou à traiter la carie dentaire ou la plaque dentaire. Il contient du caséino-glycomacropeptide (CGMP) sous forme hydrolysée, libre et de la caséine coagulée complexée avec du calcium (caséine calcifiée).

Le CGMP est un glycopeptide hydrolysé de la kappa-caséine par l'action de la présure. Il est présent dans le sérum de coagulation et n'est pas séparé lors du procédé de préparation dudit lait emprésuré. La quantité de CGMP libre, présente dans le lait emprésuré est comprise de préférence entre 0,05 et 0,2%, ce qui représente 1.5 à 6% de la matière protéique.

La caséine calcifiée est également produite par action de la présure sur la caséine. Elle a perdu sa forme micellaire comme c'est le cas pour d'autres produits laitiers frais tels que les yaourts ou les fromages acides, mais contrairement à ces derniers, elle garde une partie des propriétés de la micelle. Elle reste notamment sous une forme calcifiée. La teneur en caséine calcifiée dudit lait emprésuré est de préférence supérieure à 2%.

Le lait emprésuré selon l'invention est également riche en calcium. Ce calcium se trouve sous 2 formes : l'une associée à la caséine, et l'autre libre. La quantité minimale de calcium contenue dans le lait emprésuré est de 1,75 g/l.

Le lait emprésuré selon l'invention à la particularité de rassembler ces deux composés anti-caries : le CGMP et la caséine calcifiée sous leur forme active. Des tests réalisés *in-vivo* montrent qu'il possède des propriétés anti-cariogènes supérieures à celles d'autres produits laitiers tels que le yaourt, par exemple. On explique ce résultat surprenant par un effet de synergie entre le CGMP sous forme hydrolysée et la caséine calcifiée.

Le lait emprésuré selon l'invention peut être obtenu de différentes manière. Un moyen de le préparer est par exemple de faire coaguler du lait à pH neutre avec de la présure et ajouter un sel de calcium. Le lait emprésuré est alors stabilisé par gélification par exemple.

On peut préparer un mélange contenant au moins 80% de lait, sous forme de lait entier et/ou enrichi en matière grasse et de 1 à 10% de lait en poudre, de manière à augmenter la matière sèche. On peut ajouter du sorbate de potassium environ à raison de 0,1 % en poids, de manière à protéger le produit contre les moisissures.

Le lait entier et/ou enrichi en matière grasse peut être utilisé sous forme liquide ou poudreuse. Sa teneur en matière grasse est de préférence supérieure à 4%. On utilise de préférence un lait ayant une teneur minimale en matière sèche de 8%.

On ajoute de l'eau au mélange de manière à obtenir une pâte. On peut alors procéder à la gélification de la pâte en lui ajoutant une solution à 10% d'agar/gélatine, à une température de 50°C. L'addition se fait par dispersion à froid. On peut remplacer la gélatine par une gomme par exemple.

Le mélange peut être filtré avant d'être pasteurisé à 125°C pendant environ 20 s. Il peut être ensuite chauffé à 70°C avant d'être homogénéisé sous une pression d'environ 200 bars. Puis après refroidissement préalable du mélange jusque vers 45°C, on ajoute du chlorure de calcium en solution à 30-33% et de la présure à 1/15000, par exemple. La quantité de chlorure de calcium (CaCl₂) est de préférence comprise entre 0,.02 et 0,05 % en poids par rapport au mélange. Pour la présure, on peut l'utiliser au 1/15000 à raison de 0,005 à 0,008%. Le mélange est ensuite agité. Les conditions sont telles que le mélange à base de lait ne caille pas au cours de cette étape d'emprésurage. Pour cela la durée d'homogénéisation du mélange vers 45°C est de préférence inférieure à 30 min.

Le mélange peut être ensuite conditionné à une température de 43°C, par exemple, puis il peut être étuvé à 43°C, 1-2 heures pour caillage. On obtient alors un lait emprésuré, coagulé à pH neutre et qui contient toujours en fin de procédé une quantité de CGMP libre et de la caséine complexée avec du calcium.

Ce procédé permet donc de rassembler et de conserver d'une manière simple ces deux constituants anti-cariogènes et anti plaque dentaire sous leur forme active, la caséine sous une forme calcifiée et le CGMP hydrolysé.

Le lait emprésuré entre dans la préparation de diverses compositions alimentaires, par exemple des laits gélifiés avec des durées de conservation de 2, 3 ou 4 semaines. On peut également préparer des compositions pulvérulentes, le produit frais pouvant être dans ce cas séché par spray-drying, par exemple de manière à l'obtenir sous forme de poudre.

Ces compositions contiennent une quantité efficace dudit lait emprésuré, elles ont une activité anti-carie et anti-plaque optimalisée. Pour traiter la carie dentaire, la quantité minimale efficace de lait emprésuré est de préférence supérieure à 10%. On peut renforcer l'activité anti-caries de telles compositions en ajoutant du CGMP, par exemple.

Les compositions peuvent être édulcorées par addition de 1 à 5 % d'un édulcorant, de préférence du xylitol. On peut également utiliser de l'Aspartam, du saccharose, du tagatose ou un mélange maltitol/fructose, par exemple. Les compositions peuvent également être aromatisées.

Des tests ont été réalisés pour comparer l'effet anti-cariogène du lait emprésuré à celui de certains autres produits laitiers frais tels que par exemple le yaourt (exemple 1). Les résultats sur les effets *in vivo* ont montré de manière significative l'efficacité supérieure du lait emprésuré dans l'activité anti-cariogène.

Les exemples décrits ci-dessous ne sont pas limitatifs et servent à illustrer l'invention. Les pourcentages et les parties sont indiqués en poids sauf indication contraire.

### Exemple 1 : lait emprésuré

On prépare une pâte en mélangeant à 86 kg de lait enrichi à 5% de matière grasse et ayant une teneur en matière sèche de 8%, 7,3 kg de lait en poudre à 1 % puis 120 g de sorbate de potassium. On gélifie la pâte en ajoutant 400 g d'une solution à 10% d'agar/gélatine (200°B). L'addition se fait à une température de 50°C et par dispersion à froid. Puis on filtre le mélange avant de le pasteuriser à 125°C durant 20 s. On le chauffe ensuite à 70°C puis on l'homogénéise sous une pression de 200 bars. On laisse refroidir le mélange jusqu'à une température de 45-46°C. Puis on lui ajoute 50 g de chlorure de calcium (CaCl₂) en solution à 30-33% et 8 g de présure 1/15000. On agite le mélange pendant moins de 30 min. à 45°C.

Le mélange est ensuite étuvé à 43°C, 1-2 heures. On obtient alors un lait emprésuré, coagulé à pH neutre et qui contient en fin de procédé une quantité d'environ 0,1% de CGMP libre et environ 2% de caséine complexée avec du calcium. Ce lait emprésuré sert de base pour la préparation de diverses compositions alimentaires anti-cariogènes.

### Exemple 2 : Comparaison des effets sur la carie du rat du lait emprésuré et du yaourt nature

On prépare 3 compositions, ne contenant pas de saccharose :
- un lait emprésuré correspondant à l'invention tel que préparé selon l'exemple 1 ;
- un yaourt nature traditionnel ; et
- un témoin à base de protéine (farine) de soja (contrôle D₀).

On prépare ensuite 3 autres compositions correspondant aux précédentes additionnées de 10% de saccharose, la dernière étant le contrôle D₁.

On compare enfin l'effet anti-carie du lait emprésuré obtenu selon l'invention à celui du yaourt traditionnel, les deux produits contenant ou non 10% de saccharose.

Pour cela on prépare 6 lots de 10 rats que l'on infecte oralement 2 fois par jour en les inoculant avec des suspensions de *Streptococcus sobrinus* et *Actinomyces viscosus.* Pour favoriser l'implantation de ces bactéries, on donne aux rats de l'eau sucrée contenant 3% de glucose et 3% de sucre pendant plusieurs jours. On nourrit alors ces rats selon un programme précis réparti en 6 traitements distincts décrits dans la table 1. Tous les rats suivent un régime de base (D₂) "hautement cariogène", distribué sous forme de 18 rations journalières. On fournit aux rats en alternance 18 rations "hautement cariogènes" et 18 rations, soit des produits laitiers frais décrits ci-dessus dans les traitements 3, 4, 5 et 6, soit des contrôles D₀ et D₁ dans les traitements témoins 1 et 2 respectivement. On étudie alors les effets des traitements sur l'extension de la plaque dentaire, sur l'incidence des caries et sur la flore orale des rats.

Un lot témoin de rats suit le traitement 1 à base de protéine de soja. Les résultats de ce lot servent de référence aux traitements 3 et 4. Un second lot de rats témoin qui suivent le traitement 2 (protéines de soja + 10% de saccharose) sert de contrôle aux traitements 5 et 6.

**Table 1 : Programme - Nombre de rations distribuées pour les traitements et contrôles 1 à 6.**

| | | Traitements | **1** | 3 | 4 | **2** | 5 | 6 |
|---|---|---|---|---|---|---|---|---|
| | Diètes | | **(témoin)** | | | **(témoin)** | | |
| Nombre De Rations | 0.4g D₂ diète de base | | **x18** | x18 | x18 | **x18** | x18 | x18 |
| | 0.4g D₁ contrôle avec sucre | | | | | **x18** | | |
| | 0.4g D₀ contrôle sans sucre | | **x18** | | | | | |
| | 1 g de lait emprésuré | | | x18 | | | | |
| | 1 g de yaourt | | | | x18 | | | |
| | 1 g de lait emprésuré + 10% sucre | | | | | | x18 | |
| | 1 g yaourt + 10% sucre | | | | | | | x18 |

**Table 2: Compositions de la diète de base D₂ et des contrôles D₁ et D₀ donnés selon la Table 1.**

| | Diètes | D₂ "hautement cariogène" | D₁ "faiblement cariogène" | D₀ "non cariogène" |
|---|---|---|---|---|
| Ingrédients | | | | |
| Sucre en poudre | | 56% | 10% | - |
| "Substitut" de lait écrémé à base de farine de soja* | | 18% | 28% | 28% |
| Farine de blé | | 8% | 54% | 64% |
| Levure de bière | | 5% | 5% | 5% |
| Protéine Gevral | | 2% | 2% | 2% |
| Chlorure de sodium | | 1% | 1% | 1% |

| | | | | |
|---|---|---|---|---|
| * 43,8% extrait de soja, 54,9% lactose, 0,8% CaCl₂.2H₂O, 0.4% L-Met et 0,1% L-Lys. | | | | |

On a analysé les fissures dentaires initiales (T) et avancées (B) ainsi que les caries des surfaces lisses (E). Les résultats sont donnés dans la table 3.

**Table 3: Moyennes par rat des fissures dentaires initiales (T), fissures dentaires avancées (B) et des caries des surfaces lisses (E).**

| Traitements | T ΔΔ | B ΔΔ | E ΔΔΔ |
|---|---|---|---|
| 1 **(témoin sans sucre)** | **11,5** | **9,2** | **17,5** |
| 3 lait emprésuré | 10,3 | 6,1 | 5,4 |
| 4 yaourt | 11,3 | 7,3 | 8,3 |
| **2 (témoin avec sucre)** | **11,8** | **9,9** | **16,9** |
| 5 lait emprésuré sucré | 11,9 | 10,3 | 10,9 |
| 6 yaourt sucré | 11,7 | 9,9 | 10,9 |

| | | | |
|---|---|---|---|
| ΔΔ = 12 fissures au maximum, ΔΔΔ = 20 unités de caries au maximum. | | | |

Les résultats montrent que parmi les 2 produits laitiers frais non sucrés, seul le lait emprésuré correspondant à l'invention a fait réduire de manière significative les lésions T (traitement 3 vs 1). Le yaourt n'affecte pas de manière significative le développement de ces lésions. Lorsque ces produits sont sucrés (traitements 5, 6 vs 2), ils n'ont pas d'effet sur ces mêmes lésions.

Les résultats des tests montrent également que le lait emprésuré a une action nette et significative sur la réduction des lésions B, alors que le yaourt a une action statistiquement à la limite du significatif (traitements 3, 4 vs 1). Les produits laitiers sucrés (traitements 5, 6 vs 1) n'ont aucun effet sur ces lésions.

Pour ce qui est de l'action sur les caries des surfaces lisses (E), l'action des 2 produits laitiers frais est très significative, celle du lait emprésuré étant nettement supérieure (traitement 3 vs 1). Ces mêmes produits sucrés (traitements 5, 6 vs 2) ont un effet encore significatif.

Le lait emprésuré non sucré réduit de manière plus importante tous les types de caries que lorsqu'il est sucré (traitement 3 vs 5).

On a pu dresser le tableau récapitulatif suivant (Table 4) :

**Table 4 : Pourcentages comparés de réduction des caries (B) et (E).**

| | Fissures dentaires avancées (B) | Caries des surfaces lisses (E) |
|---|---|---|
| Témoin sans sucre | 9,2 caries = 100% | 17,5 caries = 100% |
| Yaourt nature | 21% de caries en moins | 53% de caries en moins |
| Lait emprésuré | 34% de caries en moins | 69% de caries en moins |

Les résultats montrent que ces deux produits frais ont une nette activité anti-carie. Cependant, le lait emprésuré selon l'invention qui contient du CGMP sous une forme hydrolysée libre et de la caséine calcifiée, montre une activité anti-carie nettement supérieure. Cet effet est encore plus prononcé contre les caries des surfaces lisses.

### Exemple 3

On prépare une composition pour un lait de suite en mélangeant :

| | |
|---|---|
| Lait emprésuré préparé selon l'exemple 1 | 10,0%, |
| Poudre de lactosérum déminéralisé contenant 12,5% de protéines | 64,9% |
| Matière grasse du lait | 20,6% |
| Huile de maïs | 4,4% |
| K₂HPO₄ | 0,1% |

On obtient alors une composition pour une formule infantile qui a des propriétés anti-cariogènes.

### Exemple 4

On prépare une composition anti-cariogène pour l'alimentation infantile. Pour cela on mélange :

| | |
|---|---|
| Lait emprésuré préparé selon l'exemple 1 | 12,6% |
| Poudre de lactosérum déminéralisé contenant 12,5% de protéines | 22,9% |
| Lactose | 34,9% |
| Matière grasse du lait | 20,4% |
| Huile de maïs | 4,6% |
| Minéraux, micronutriments et eau | 4,6% |

### Exemple 5

On prépare une composition pour une boisson lactée ayant une activité anti-caries ou anti- plaque dentaire, en mélangeant les ingrédients suivants :

| | |
|---|---|
| Lait emprésuré préparé selon l'exemple 1 | 10-15 g |
| Lactose | 5-10 g |
| Eau | 70-90 g |

La somme de ces composants devant donner 100g.

### Exemple 6

On ajoute au lait emprésuré préparé selon l'exemple 1, 1 à 2% de variacine sous forme de poudre de bactériocines tel que décrite dans le brevet EP 0 759 469.

La composition réfrigérée obtenue a les propriétés anti-cariogènes du lait emprésuré et une durée de conservation allongée.

### Exemple 7

On prépare à partir du lait emprésuré obtenu à l'exemple 2, une composition alimentaire réfrigérée, édulcorée et aromatisée. Pour ce faire, on ajoute à 100g de lait emprésuré obtenu selon l'exemple 2, 5g de xylitol et 2g d'un agent aromatisant.

La composition obtenue est un produit laitier frais ayant un agréable goût sucré et une activité anti-cariogène optimalisée.

### Exemple 8

On peut préparer une composition alimentaire réfrigérée, édulcorée et aromatisée à partir des ingrédients suivants:

| | |
|---|---|
| Lait écrémé | 78-82% |
| Crème à 40% de MG | 10-12% |
| Poudre de lait écrémé | 6-8% |
| Agar/gélatine | 0.4-2% |
| Sorbate de potassium | 0.05-0.2% |
| Aspartam/Acesulfame | 0.02-0.06% |
| Arômes/colorants (fraise,banane,etc) | 0.05-0.2% |
| Total | 100 % |

On chauffe le mélange à 70°C, puis on l'homogénéise sous une pression de 200 bars. On pasteurise à 110°C 6 min, puis on refroidit à 43°C. On emprésure avec environ 4 g de présure au 1/15000 pour 100kg de mélange. Le produit est conditionné puis coagulé pendant environ 1h 30 mn puis réfroidit à 4°C.
On peut préparer une variante en utilisant comme source de sucre environ 7.5% du mélange suivant : 1:4 à 4:1 de maltitol et 4:1 à 1:4 de fructose. Pour ne pas dépasser 100%, on diminue la quantité de lait écrémé.

La composition obtenue est un produit laitier frais ayant un agréable goût sucré et une activité anti-cariogène et anti plaque optimalisée. Elle contient au minimum 0,05% de CGMP sous une forme active et minimum 2% de caseine complexée.

### Exemple 9

On peut préparer un lait emprésuré brassé à partir du mélange décrit à l'exemple 8 mais après refroidissement à 4°C, on ajoute environ 10% de fromage frais ou de yaourt (pour ne pas dépasser 100%, on diminue la quantité de lait écrémé), puis on brasse le mélange, on le lisse et on le conditionne.
On obtient un produit laitier frais aromatisé brassé, ayant une activité anti-carie et anti-plaque.

### Exemple 10

On peut préparer une composition alimentaire réfrigérée ayant une activité anti-caries et anti-plaque optimalisée, à partir des ingrédients suivants:

| | |
|---|---|
| Lait écrémé | 30-35% |
| Crème à 40%de MG | 27% |
| Poudre de lait écrémé | 3-5% |
| Amidon | 2-4% |
| Maltitol/fructose | 15-18% |
| Cacao en poudre | 2% |
| Fromage frais | 10% |

On ajoute du citrate trisodique et des arômes, puis on complète à 100% en ajoutant de l'eau.

On chauffe à 110°C pendant environ 2 minutes le mélange lait-crème-poudre de lait, puis on le refroidit à 43°C. On emprésure avec environ 4 g %kg de mélange pendant 1h30. On chauffe à 85°C puis on ajoute les autres ingrédients. On homogénéise sous pression et on traite à 130°C pendant environ 40 secondes et on conditionne après refroidissement.

### Exemple 11 composition anti-carie en poudre

On pasteurise du lait (écrémé le cas échéant) puis on le refroidit entre 10 et 40°C avant d'ajouter de la présure à raison d'environ 0.1 % puis on laisse agir de 60 à 600 min. On homogénéise sous pression puis on ajoute les graisses. On chauffe à 70°C, on homogénéise de nouveau puis on chauffe à 120°C. On concentre à environ 50% et on sèche le mélange (spray drying) après homogénéisation.

La composition en poudre ainsi obtenue a les mêmes propriétés anti-caries et anti-plaque que les produits laitiers frais selon l'invention. Les quantités de CGMP et de caseine sous leurs formes actives sont telles que décrites précédemment dans la description.

## Revendications

1. Utilisation d'un lait emprésuré qui contient à la fois du caséino-glycomacropeptide (CGMP) sous forme hydrolysée et de la caséine coagulée, complexée avec du calcium, ces 2 composés étant sous leur forme active, pour préparer des compositions alimentaires destinées à prévenir ou à traiter la carie dentaire ou la plaque dentaire.

2. Utilisation selon la revendication 1, dans laquelle la quantité minimale de CGMP présente dans le lait emprésuré est de 0,05% en poids du total.

3. Utilisation selon la revendication 1 ou 2, dans laquelle la quantité minimale de caséine complexée présente dans le lait emprésuré est de 2% en poids du total.

4. Utilisation selon l'une des revendications 1 à 3, dans laquelle la quantité minimale efficace de lait emprésuré est de 10% en poids.

5. Utilisation selon l'une des revendications 1 à 4, dans laquelle la composition alimentaire est sous forme de poudre.

6. Composition alimentaire anti-cariogène destinée à prévenir ou à traiter la carie dentaire ou la plaque dentaire, comprenant du lait emprésuré qui contient à la fois du caséino-glycomacropeptide (CGMP) sous forme hydrolysée et de la caséine coagulée, complexée avec du calcium, ces 2 composés étant sous leur forme active.

7. Composition alimentaire selon la revendication 6, dans laquelle la quantité minimale de CGMP présente dans le lait emprésuré est de 0,05% en poids du total.

8. Composition alimentaire selon la revendication 6 ou 7, dans laquelle la quantité minimale de caséine complexée présente dans le lait emprésuré est de 2% en poids du total.

9. Composition alimentaire selon l'une des revendications 6 à 8 dans laquelle du CGMP est rajouté de manière à renforcer l'activité anti-caries et anti-plaque de cette composition.

10. Composition alimentaire selon l'une des revendications 6 à 9 dans laquelle la quantité minimale efficace de lait emprésuré est de 10% en poids.

11. Composition alimentaire selon l'une des revendications 6 à 10 présentée sous forme de poudre.

## Claims

1. Use of a renneted milk which contains at the same time caseinoglycomacropeptide (CGMP) in the hydrolysed form and coagulated casein, complexed with calcium, these two compounds being in their active form, for preparing food compositions intended to prevent or to treat dental caries or dental plaque.

2. Use according to claim 1, wherein the minimum quantity of CGMP present in the renneted milk is 0.05% by weight of the total.

3. Use according to claim 1 or 2, wherein the minimum quantity of complexed casein present in the renneted milk is 2% by weight of the total.

4. Use according to one of claims 1 to 3, wherein the minimum effective quantity of renneted milk is 10% by weight.

5. Use according to one of claims 1 to 4, wherein the food composition is in powder form.

6. Anti-caries food composition intended to prevent or to treat dental caries or dental plaque, comprising a renneted milk which contains at the same time caseinoglycomacropeptide (CGMP) in the hydrolysed form and coagulated casein, complexed with calcium, these two compounds being in their active form.

7. Food composition according to claim 6, wherein the minimum quantity of CGMP present in the renneted milk is 0.05% by weight of the total.

8. Food composition according to claim 6 or 7, wherein the minimum quantity of complexed casein present in the renneted milk is 2% by weight of the total.

9. Food composition according to one of claims 6 to 8, wherein CGMP is added in order to reinforce the anti-caries or anti-plaque activity of this composition.

10. Food composition according to one of claims 6 to 9, wherein the minimum effective quantity of renneted milk is 10% by weight.

11. Food composition according to one of claims 6 to 10 presented in powder form.

## Patentansprüche

1. Verwendung von mit Lab versetzter Milch, die gleichzeitig Casein-Glycomakropeptid (CGMP) in hydrolysierter Form und mit Calcium komplexiertes koaguliertes Casein enthält, wobei diese beiden Verbindungen sich in ihrer aktiven Form befinden, zur Herstellung von Nahrungsmittelzusammensetzungen, die zur Vorbeugung oder Behandlung der Zahnkaries oder der Zahnplaque bestimmt sind.

2. Verwendung nach Anspruch 1, bei der die Mindestmenge an CGMP, die in der mit Lab versetzten Milch vorhanden ist, 0,05 % vom Gesamtgewicht beträgt.

3. Verwendung nach Anspruch 1 oder 2, bei der die Mindestmenge an komplexiertem Casein, die in der mit Lab versetzten Milch vorhanden ist, 2 % vom Gesamtgewicht beträgt.

4. Verwendung nach einem der Ansprüche 1 bis 3, bei der die wirksame Mindestmenge an mit Lab versetzter Milch 10 Gew.-% beträgt.

5. Verwendung nach einem der Ansprüche 1 bis 4, bei der die Nahrungsmittelzusammensetzung in Pulverform vorliegt.

6. Nahrungsmittelzusammensetzung, die zur Vorbeugung oder Behandlung der Zahnkaries oder der Zahnplaque bestimmt ist und mit Lab versetzte Milch umfasst, die gleichzeitig Casein-Glycomakropeptid (CGMP) in hydrolysierter Form und mit Calcium komplexiertes koaguliertes Casein enthält, wobei diese beiden Verbindungen sich in ihrer aktiven Form befinden.

7. Nahrungsmittelzusammensetzung nach Anspruch 6, bei der die Mindestmenge an CGMP, die in der mit Lab versetzten Milch vorhanden ist, 0,05 % vom Gesamtgewicht beträgt.

8. Nahrungsmittelzusammensetzung nach Anspruch 6 oder 7, bei der die Mindestmenge an komplexiertem Casein, die in der mit Lab versetzten Milch vorhanden ist, 2 % vom Gesamtgewicht beträgt.

9. Nahrungsmittelzusammensetzung nach einem der Ansprüche 6 bis 8, bei der CGMP so zugesetzt wird, dass die Antikaries- und Antiplaqueaktivität dieser Zusammensetzung verstärkt wird.

10. Nahrungsmittelzusammensetzung nach einem der Ansprüche 6 bis 9, bei der die wirksame Mindestmenge an mit Lab versetzter Milch 10 Gew.-% beträgt.

11. Nahrungsmittelzusammensetzung nach einem der Ansprüche 6 bis 10 in Pulverform.
